# EUROPEAN PATENT APPLICATION

(11) **EP 4 129 903 A1**
(43) Date of publication of application: **08.02.2023**
(21) Application number: 21781469.8
(22) Date of filing: 29.01.2021
(51) Int. Cl.: C01B 33/02, A23K 20/20, A23K 50/40, A23K 50/80, A23L 3/358, A23L 5/00, A23L 29/00, A61K 8/19, A61K 8/25, A61K 9/06, A61K 9/107, A61K 9/12, A61K 9/16, A61K 33/00, A61K 47/02, A61K 47/04, A61Q 19/00

(54) **COMPOSITE MATERIAL**

(30) Priority: 02.04.2020 JP 2020066999
(71) Applicant: BOSQUET SILICON CORP., Osaka-shi, Osaka 530-0001 (JP); KIT Co., Ltd., Kita-ku Osaka-shi Osaka 530-0001 (JP); Mitsui Mining & Smelting Co., Ltd., Shinagawa-ku Tokyo 141-8584 (JP)
(72) Inventor: KOBAYASHI, Yuki, Kyoto-shi, Kyoto 605-0981 (JP)
(74) Representative: Moré, Solveig Helga
(86) International application number: PCT/JP2021/003383
(87) International publication number: WO 2021/199644

(57) **Abstract**

A composite material 100 of the present invention includes silicon particles or silicon fine particles 10 capable of generating hydrogen, and a metal element (iron (Fe)) 20 supported on, or attached or adsorbed to at least a part of surfaces of the silicon particles or silicon fine particles 10, or chemically bonded to the surfaces. According to the composite material 100, it is possible to exhibit a capability of generating hydrogen much higher than that of silicon particles or silicon fine particles 10 on which the metal element 20 is hardly supported or to which the metal element 20 is hardly attached or adsorbed, or silicon particles or silicon fine particles 10 which are hardly chemically bonded to the metal element.

## Description

### TECHNICAL FIELD

The present invention relates to a composite material, a hydrogen supply material, a medicament, a quasi-pharmaceutical product, a hydrogen supply material, a feed, a supplement, a food additive and a food product containing the composite material, and a formulation, a fresh food product, a cosmetic or perfumery.

### BACKGROUND ART

Hydrogen is widely applied, and expected to be used in many applications. Examples thereof include targets caused by oxidative stress, and published involvement in growth. For example, in bodies of animals including humans, active oxygen produced in derived from oxygen generated in mitochondria in cells by metabolism in the body and subdermally under ultraviolet irradiation, and derived from oxygen taken in from the lung is present. Active oxygen is known to oxidize and damage cells that form a living body while it is necessary for life support. Particularly a hydroxyl radical which has the strongest oxidizing power in active oxygen is considered to cause various diseases such as cancer, stroke, myocardial infarction, diabetes, other lifestyle diseases, and skin disorders such as skin aging and dermatitis. Therefore, it is desirable that excess active oxygen, particularly a hydroxyl radical, which has not been used in a reaction useful for a living body, be prevented from being present in the body wherever possible.

Regarding exclusively oxidative stress in a living body, hydroxyl radicals produced in the body are eliminated by reacting with some substances. Antioxidants in a living body, such as polyphenol, vitamin C, α-tocopherol or glutathione are generally considered as examples of substances that eliminate hydroxyl radicals. However, these substances eliminate not only hydroxyl radicals but also active oxygen having a function in the body, such as hydrogen peroxide, and thus may have adverse effects (side effects) such as a decrease in immunity. In addition, it is known that hydrogen can also eliminate hydroxyl radicals. However, hydrogen reacts only with hydroxyl radicals in active oxygen, and therefore does not have the above-described adverse effects (side effects). Thus, an apparatus for producing hydrogen water containing hydrogen that eliminates hydroxyl radicals in the body has been proposed (for example, Patent Document 1).

However, hydrogen (H₂) has an extremely low saturated solubility of 1.6 ppm in water at 25°C, and hydrogen in hydrogen water is easily diffused into air. Thus, for taking hydrogen in the body in an amount necessary for eliminating hydroxyl radicals, it is necessary that the concentration of dissolved hydrogen in hydrogen water be kept high. Therefore, with a method in which hydrogen water is ingested, it is impossible to take hydrogen in the body in an amount sufficient to react the hydrogen with hydroxyl radicals in the body. Thus, for easily taking hydrogen in the body, a hydrogen-containing composition containing hydrogen and a surfactant has been proposed (Patent Document 2), but it is not possible to keep the hydrogen concentration high in the body over a long period of time. Hydroxyl radicals have high reactivity, rapidly oxidize cells, and are constantly generated in a living body, and thus it is necessary to constantly supply high-concentration hydrogen into the body for eliminating the hydroxy radicals.

In view of the above-described background, a solid preparation has been disclosed which contains silicon fine particles as a main component, has high capability of generating hydrogen, and can be intended for oral use (Patent Document 3). In addition, a composite material has been disclosed which contains silicon fine particles, silicon suboxide (SiOx, where x is 1/2, 1, and 3/2) covering at least a part of surfaces of the silicon fine particles and/or a mixed composition of the silicon suboxide and silicon dioxide (Patent Document 4).

### PRIOR ART DOCUMENTS

### PATENT DOCUMENTS

Patent Document 1: Japanese Patent No. 5514140
Patent Document 2: Japanese Patent Laid-open Publication No. 2015-113331
Patent Document 3: International Publication WO 2017/130709
Patent Document 4: International Publication WO 2019/211960
Patent Document 5: International Publication WO 2018/037752
Patent Document 6: International Publication WO 2018/037818
Patent Document 7: International Publication WO 2018/037819

### NON-PATENT DOCUMENTS

Non-Patent Document 1: Matsuda et al., Water decomposition due to silicon nanoparticles and hydrogen concentrations, Extended Abstracts of the 62nd JSAP Spring Meeting, 2015, 12-031
Non-Patent Document 2: Fujinoe et al., Generation of hydrogen by reaction of silicon nanoparticles with neutral region, Extended Abstracts of the 64th JSAP Spring Meeting, 2015, 15a-421-6

### SUMMARY OF THE INVENTION

### PROBLEMS TO BE SOLVED BY THE INVENTION

However, even when hydrogen water is ingested, the amount of hydrogen contained in 1 liter of hydrogen water kept at 25°C is only 18 ml in terms of a gas. In addition, since hydrogen is the smallest molecule, is light, and has a high diffusion rate, it is impossible to thoroughly store hydrogen in a container containing the hydrogen water. A case of use in a living body will be described as an example. In the stomach, most of hydrogen in hydrogen water is gasified. Thus, there is the problem of causing pneumophagia (so-called "burp") because a sufficient amount of hydrogen is not taken in the body. Therefore, according to known information, it is impossible to keep the hydrogen concentration high in the body over a long period of time by ingesting hydrogen water and to ingest hydrogen water intermittently many times on a consecutive basis over a long period of time. On the other hand, when a hydrogen-containing composition with hydrogen encapsulated by a surfactant is ingested, it is impossible to take a sufficient amount of hydrogen in the body over a long period of time by ingesting the hydrogen-containing composition. In addition, there may arise the above-mentioned problem that hydrogen is released in the stomach. Further, assume that the hydrogen is used on the skin. Since use on the skin is no different from use in the air, hydrogen diffuses into the air and scatters in a short time because it diffuses at a much higher rate than in the body. Therefore absorption from the skin is difficult. While the situation in a living body has been described here, there are various other problems in realization of practical use in terms of industrial use, use for plants or use for energy. Further, regarding generation of hydrogen using the silicon fine particles which have been disclosed heretofore, there is still room for improvement in at least the amount of generation and the rate of generation of the hydrogen.

### SOLUTIONS TO THE PROBLEMS

The present invention solves at least one of the technical problems described above, and can significantly contribute to more intense exhibition of the capability of generating hydrogen (production ability) of silicon particles or silicon fine particles. For example, it is possible to significantly contribute to rapidly generating a large amount of hydrogen depending on a situation or more reliably extracting a large amount of hydrogen in a human or animal body, or in various moisture-containing spaces or water-containing liquids.

The present inventor has extensively conducted studies and analyses in order to find a substance capable of generating hydrogen surpassing that of the capability of generating hydrogen of the silicon fine particles which have been heretofore disclosed. Regarding the generation of hydrogen, it is required to design a substance particularly in consideration of the rate of generation of hydrogen in the initial stage among the total amount of generation (total amount of production) and the amount of generation of hydrogen per unit time (i.e. rate of generation of hydrogen). Thus, while regarding silicon (Si) as one of elements, the present inventor has conducted studies including those on effects of other elements for enhancing the capability of generating hydrogen. In addition, for silicon (Si), not only "silicon fine particles" including, as main particles, particles having an average crystallite diameter at a micron level or smaller, specifically a crystallite diameter of 1 nm or more and less than 1 µm, but also "silicon particles" having an average crystallite diameter of 1 µm or more have been covered, and studies and analyses have been conducted for finding a substance capable of exhibiting a high capability of generating hydrogen.

In the process of the experiments and analyses, the present inventor has analyzed the capability of generating hydrogen of a composite material in which a specific metal element is supported on or attached or adsorbed to at least a part of surfaces of silicon particles or silicon fine particles (hereinafter, also referred to collectively as "silicon particles"), or the surfaces are chemically bonded to the metal, and resultantly, the present inventor has found that the composite material more intensely exhibits the capability of generating hydrogen. More specifically, the present inventor has found that the composite material is considerably superior in the total amount of generation (total amount of production) of hydrogen and/or the amount of generation of hydrogen per unit time in the initial stage to conventional silicon fine particles. In the present application, the "surface" of a silicon particle means the surface of a silicon suboxide film covering at least a part of the surface of the silicon fine particle or the silicon particle when the silicon particle includes the silicon suboxide film, the surface of a film of mixed composition of the silicon suboxide and silicon dioxide when the silicon particle includes the surface of the film of the mixed composition, or the surface of a natural oxide film when the silicon particle includes the natural oxide film.

In addition, the present inventor has found that employment of the composite material increases the amount of generation of hydrogen, and resultantly, after the hydrogen generation reaction, the thickness of silicon suboxide (SiOx, where x is 1/2, 1, and 3/2; the same applies hereinafter) covering at least a part of surfaces of the silicon particles or the like and/or the thickness of the mixed composition of the silicon suboxide and silicon dioxide are larger than the thickness for conventional silicon fine particles.

The present invention has been made on the basis of the above-mentioned points of viewpoint.

The "silicon fine particles" in the present application include, as main particles, particles having an average crystallite diameter of 1 nm or more and less than 1 µm. In a narrower sense, the "silicon fine particles" in the present application include, as main particles, silicon nanoparticles having an average crystallite diameter at a nano level, specifically a crystalline diameter of 1 nm or more and 500 nm or less. In addition, the "silicon particles" in the present application include, as main particles, particles having an average crystallite diameter of more than 500 nm (in a narrow sense, 1 µm or more) and 500 µm or less.

In addition, in the present application, the "silicon fine particles" include not only those in which silicon fine particles are dispersed, but also those in which a plurality of silicon fine particles are aggregated to form aggregates in a µm order (generally 0.1 µm or more). Each of the above-described numerical value ranges for "silicon fine particles" is merely an example, and the numerical value range is not limited. In addition, the crystallite diameter is appropriately selected depending on the application, use method, required function and the like of the "silicon fine particles" or the "silicon particles".

In addition, the "water-containing liquid" in the present application is water or an aqueous solution, and encompasses, for example, gastrointestinal tract internal fluid of animals (including humans). The "gastrointestinal tract internal fluid" refers to small intestine internal fluid and large intestine internal fluid. It goes without saying that the example of the "water-containing liquid" is not limited to the above-described example. In addition, the material of the "pH adjusting agent" (hereinafter, referred to as an "alkali agent") in the present application is not particularly limited as long as it is an agent capable of adjusting the pH value to fall within an alkali range of more than 7 (typically more than 7.4). In addition, use on the skins of animals (including humans) is included. When the composite material is used as an active oxygen neutralizing agent in a living body, it is preferable to use an alkali agent approved as any of a medicament (Pharmacopoeia drug), a quasi-pharmaceutical product and a food additive. As described above, the composite material is not limited to that for animals (including humans). Examples of the alkali agent that can be employed include sodium hydrogencarbonate, sodium carbonate, sodium dihydrogen phosphate, disodium hydrogenphosphate, potassium hydrogencarbonate, potassium carbonate, and pH adjusting agents for medicaments, quasi-pharmaceutical products, food products, or cosmetics. Sodium hydrogencarbonate which is the most versatile of the above-mentioned alkali agents is widely used for medicaments, quasi-pharmaceutical products or food additives because sodium hydrogencarbonate has a plurality of advantages such that it has a pH value adjustment function required in the present invention, and is excellent in safety and versatility. On the other hand, in industrial applications, the pH adjusting agent is not limited to those described above, and a wide range of pH adjusting agents can be employed. For any of the pH adjusting agents, a form in which the pH adjusting agent is not decomposed by an acid is a preferred aspect. In particular, when the composite material of the present application is orally ingested, a form is preferable in which composite material is not decomposed or is hardly decomposed by gastric acid.

A composite material of the present invention includes silicon particles or silicon fine particles capable of generating hydrogen, and a physiologically acceptable or medically acceptable metal element supported on, or attached or adsorbed to at least a part of surfaces of the silicon particles or silicon fine particles, or chemically bonded to the surfaces.

Detailed mechanisms is still unknown, but in this composite material, support, attachment or adsorption (hereinafter, referred to collectively as "support") of a metal element (or a metal element-containing substance; hereinafter, referred to collectively as a "metal element") increases the amount of generation of hydrogen due to the catalytic action of the metal element, and as a result, the thickness of silicon suboxide covering at least a part of silicon fine particles or silicon particles and/or the thickness of a mixed composition of the silicon suboxide and silicon dioxide can be made larger than the thickness for conventional silicon fine particles. That is, by employing this composite material, a reaction can more reliably occur in which silicon and water (or water in a water-containing liquid) are brought into contact with each other to form hydrogen, thereby forming silicon oxide. As a result, it is possible to exhibit a capability of generating hydrogen much higher than that of silicon particles or silicon fine particles on which the metal element is hardly supported (or not supported) or silicon particles or silicon fine particles which are hardly chemically bonded (or not chemically bonded) to the metal element. More specifically, this composite material is considerably superior in the total amount of generation (total amount of production) of hydrogen and/or the amount of generation of hydrogen (the amount of production of hydrogen) per unit time in the initial stage to conventional silicon fine particles. In particular, it is worth noting that the capability of generating hydrogen is intensely exhibited even in silicon particles having a crystallite diameter of 1 µm or more, which are relatively large particles.

### EFFECTS OF THE INVENTION

A composite material of the present invention is excellent in the total amount of generation (total amount of production) of hydrogen and/or the amount of generation of hydrogen per unit time in the initial stage.

### BRIEF DESCRIPTION OF THE DRAWINGS

Fig. 1 is a schematic view of a composite material according to a first embodiment.
Fig. 2 is a graph showing a temporal change of hydrogen generated by reactions of composite materials of Exampled 1 and 2 and a comparative example with a water-containing liquid.
Fig. 3 shows cross-sectional TEM images showing silicon oxide thicknesses for a composite material (a) of Example 1 and a comparative example (b).
Fig. 4(a) is a side view showing a laminated structure of a layered body and a medium before hydrogen is generated in a second embodiment, and Fig. 4(b) is a side view showing a laminated structure of a layered body and a medium when hydrogen is generated in the second embodiment.
Fig. 5 is a schematic view showing a foam containing or provided with a composite material in a second embodiment.
Fig. 6 is a side view showing a layered body structure in a modification (1) of the second embodiment.

### EMBODIMENTS OF THE INVENTION

Embodiments of the present invention will be described on the basis of the accompanying drawings. In this description, like parts are given like reference symbols throughout the drawings unless otherwise specified. Further, in the drawings, elements of the embodiments are not necessarily shown on an actual scale. In addition, some reference symbols may be omitted for facilitating visualization of the drawings.

### [1] Composite material and method for production thereof

### FIRST EMBODIMENT

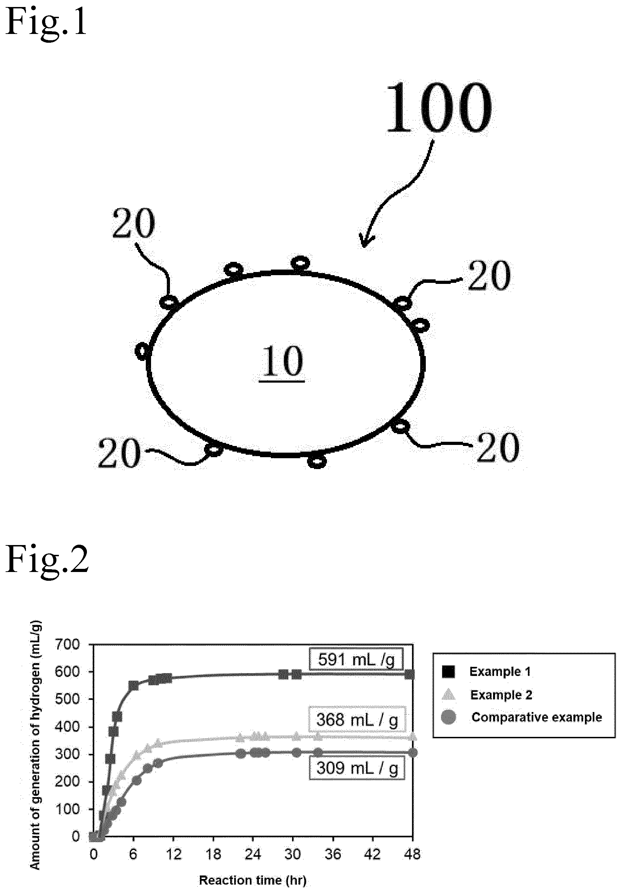

A composite material 100 according to the present embodiment, and a method for producing the composite material 100 will be described in detail. Fig. 1 is a schematic view of the composite material 100 according to the present embodiment. As shown in Fig. 1, the composite material 100 according to the present embodiment includes silicon particles or silicon fine particles (hereinafter, also referred to collectively as "silicon particles") 10 capable of generating hydrogen, and a physiologically acceptable or medically acceptable metal element 20 (hereinafter, referred to collectively as a "physiologically acceptable metal element 20") supported on, or attached or adsorbed to at least a part of surfaces of the silicon particles 10, or chemically bonded to the surfaces.

In the present embodiment, the metal element 20 is iron (Fe). The metal element 20 supported on, or attached or adsorbed to at least a part of the silicon particles 10 is an example of an aspect that can be employed by the composite material 100 according to the present embodiment. On the other hand, the state in which a part of the metal element 20 and the surfaces of the silicon particles 10 are chemically adsorbed and the state in which a compound (e.g. silicide) is formed in a region where the metal element 20 is in contact with the surfaces of the silicon particles 10 are an example in which the surfaces of the silicon particles 10 are chemically bonded to the metal element 20.

In addition, the amount of the metal element 20 supported on at least a part of the surfaces of the silicon particles 10 or chemically bonded to the surfaces is not particularly limited. The typical amount of the metal element 20 is 0.1 ppmw or more and 1000 ppmw or less in terms of a mass ratio. From the viewpoint of obtaining a high capability of generating hydrogen including that in the initial stage as described later, the lower limit of the numerical value range in the mass ratio of the amount of the metal element 20 is preferably 0.5 ppmw or more, more preferably 1 ppmw or more, still more preferably 1.5 ppmw or more, and even more preferably 2 ppmw or more. The upper limit of the numerical value range is preferably 500 ppmw or less, more preferably 300 ppmw or less, still more preferably 100 ppmw or less, even more preferably 75 ppmw or less, furthermore preferably 50 ppmw or less.

### [Method for producing composite material 100]

Next, the method for producing the composite material 100 according to the present embodiment. In addition, a surface of the composite material 100 measured or observed in the process of reaction of the composite material 100 with a water-containing liquid, and a silicon oxide film covering the surface will be described.

### <Pulverization step>

In the present embodiment, for example, commercially available high-purity silicon particle powder (particle size: 300 µm or less, purity: 99.999%, i-type silicon) is used as a part of raw materials for the composite material 100. In another aspect of the present embodiment, silicon particle powder having a purity higher than or lower than the aforementioned purity can be employed.

First, the high-purity silicon particle powder is subjected to pulverization treatment (first pulverization step) by a jet mill method using a cutter. A modification of the present embodiment in which after the first pulverization step, an additional pulverization step (second pulverization step) using zirconia beads of 0.5 mmϕ in ethanol is carried out in a bead mill method to obtain silicon fine particles (silicon nanoparticles) 10 having a representative crystallite diameter of less than 500 nm is also an aspect that can be employed. Further, an aspect in which instead of the first pulverization step, a roller mill method, a high-speed rotation pulverization method or a container driving mill method is used to obtain silicon particles 10 having a crystallite diameter of 1 µm or more and 60 µm or less or silicon fine particles (silicon nanoparticles) 10 having a representative crystallite diameter of less than 500 nm is an aspect that can be employed.

Employment of a mixed solution of ethanol at 99% or more (e.g. 99.5 wt%) and a small amount of water (e.g. 0.1 wt% or more and 10 wt% or less, more preferably more than 1 wt% and 2 wt% or less) in the case performing wet treatment in each of the pulverization steps is a preferred aspect from the viewpoint of enhancing the reliability of safety (e.g. safety for human bodies) of the finally produced composite material 100 containing silicon particles 10.

### <Classification step>

Carrying out a classification step between the pulverization step (first pulverization step or second pulverization step) in the present embodiment and a metal element supporting step as described later is also an aspect of the present embodiment. Specifically, substantially all of silicon fine particles 10 having a crystallite diameter of less than 1 µm and aggregates of the silicon fine particles 10 having a crystallite diameter of less than 1 µm are removed using an airflow method so that the ratio of silicon fine particles 10 having a crystallite diameter of less than 1 µm and aggregates of the silicon fine particles 10 having a crystallite diameter of less than 1 µm to all the silicon particles 10, the silicon fine particles 10 and the aggregates thereof is 5 mass% or less (more preferably 3 mass% or less, still more preferably 1 mass% or less, still more preferably 0.5 mass% or less, even more preferably 0.2 mass% or less). The term "crystallite diameter" is employed for the silicon particle 10, whereas for the aggregate of silicon particles 10, the term "crystal grain diameter" which means the diameter of the entire aggregate is used.

When the composite material 100 capable of generating hydrogen in the present embodiment contains silicon particles 10 and/or silicon fine particles 10, and the ratio of silicon fine particles 10 having a crystallite diameter of less than 1 µm and aggregates of the silicon fine particles 10 having a crystallite diameter of less than 1 µm to all the silicon particles 10, the silicon fine particles 10 and the aggregates thereof is 5 mass% or less (more preferably 3 mass% or less, still more preferably 1 mass% or less, still more preferably 0.5 mass% or less, even more preferably 0.2 mass% or less), an effect can be exhibited such that safety can be enhanced by more reliably preventing passage of the composite material 100 through the cell membranes and between cells of the intestinal tract.

The upper limit of the crystallite diameter of silicon particles 10 and aggregates of silicon particles 10 is not limited as long as they have a capability of generating hydrogen. However, in addition to the classification step described above, a classification step carried out to remove substantially all of silicon particles 10 having a crystallite diameter of more than 60 µm or more preferably more than 45 µm and aggregates of the silicon particles 10 can be employed. More specifically, a classification step of performing classification so that the ratio of the silicon particles having a crystallite diameter of more than 60 µm (more preferably more than 45 µm) and aggregates of the silicon fine particles to all the silicon particles and the aggregates is 5 mass% or less (more preferably 3 mass% or less, still more preferably 1 mass% or less, even more preferably 0.5 mass% or less, even more preferably 0.2 mass% or less) can be employed. By removing substantially all of silicon particles 10 having a crystallite diameter of more than 60 µm or more preferably more than 45 µm and aggregates of the silicon particles 10, the capability of generating hydrogen can be kept high, and a more stable capability of generating hydrogen can be exhibited because the crystallite diameter falls within a certain range. In addition, in the case of oral ingestion, deterioration of human mouthfeel can be suppressed. In addition, it is possible to reliably prevent silicon particles from being directly absorbed and entering the blood vessel.

Resultantly, as an example, silicon particles 10 and aggregates of silicon particles 10 are obtained in which the ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less (more preferably 3 mass% or less, still more preferably 1 mass% or less, still more preferably 0.5 mass% or less, even more preferably 0.2 mass% or less). In another aspect, silicon particles 10 having a crystallite diameter of 1 µm or more and 60 µm or less (more preferably 1 µm or more and less than 45 µm) and aggregates of the silicon particles 10 is obtained.

As described above, a process can be employed in which a classification step is not carried out, and a metal element supporting step as described later is carried out, and as described above, it is preferable to carry out any of the above-described classification steps from the viewpoint of achieving a stable capability of generating hydrogen, suppressing deterioration of human mouthfeel upon oral ingestion, or reducing the possibility of ingress of silicon particles into the blood vessel.

### <Step of supporting metal element>

Next, a step of supporting the metal element 20 on silicon particles 10 or attaching or adsorbing the metal element 20 to silicon particles 10, or a chemical bonding step of chemically bonding the metal element 20 and the surfaces of silicon particles 10 (hereinafter, referred to collectively as a "supporting step") is carried out.

In an example of the supporting step in the present embodiment, spraying treatment using a spray apparatus for spraying a solution (e.g. an aqueous chloride solution) of the metal element 20 (e.g. iron (Fe)) to the silicon particles 10 that is being stirred is performed in a treatment chamber. For example, a spraying step of spraying a 10 mM aqueous FeCl₂ solution to silicon particles 10 is carried out. The solution of the metal element 20 is a part of raw materials for the composite material 100.

In another example of the supporting step in the present embodiment, another step of bringing the silicon particles 10 into contact with the solution of the metal element 20 can be employed instead of the spraying step or together with the spraying step. As a specific example of the other step, a step of immersing silicon particles 10 in the solution of the metal element 20 manually or automatically by using a storage portion for storing the solution of the metal element 20 can be employed.

In another example of the supporting step in the present embodiment, a step of performing pulverization treatment using a SUS cutter in the pulverization step, whereby for example iron (Fe) is supported on or attached or adsorbed to silicon particles 10 with the utilization of the composition of the cutter in the pulverization process. In that case, the pulverization step described above can also play a role as the supporting step in the present embodiment.

### <Analysis on generation of hydrogen by contact between composite material 100 and water-containing liquid>

According to the present inventor, the composite material 100 is then immersed in a water-containing liquid (pH value: 8.2) at a temperature equivalent to the human body temperature (37°C) which is an aqueous solution having sodium hydrogencarbonate as a pH adjusting agent as a solute in order to analyze the total amount of generation (total amount of production) of hydrogen (H₂) and the rate of generation (rate of production) of hydrogen when a contact step of bringing the water-containing liquid and the composite material 100 into contact with each other is carried out.

### <Examples>

Hereinafter, the first embodiment will be described more in detail by way of examples, but the first embodiment is not limited to these examples.

### [Example 1]

Example 1 is a composite material 100 in which a part of surfaces of silicon particles 10 support a metal element 20, or the surfaces are chemically bonded to the metal element 20. The metal element 20 in Example 1 is iron (Fe). In Example 1, the mass ratio of the metal element 20 to silicon particles 10 is about 10 ppmw.

### [Example 2]

Like Example 1, Example 2 is a composite material 100 in which a part of surfaces of silicon particles 10 support a metal element 20, or the surfaces are chemically bonded to the metal element 20. The metal element 20 in Example 2 is iron (Fe). In Example 2, the mass ratio of the metal element 20 to silicon particles 10 is about 5 ppmw.

As a comparative example, silicon particles produced through steps identical to those in Example 1 except that the supporting step in the first embodiment was not carried out were employed. The mass ratio of the metal to the silicon particles in the comparative example is less than 0.1 ppmw, and in a more narrow sense, the mass ratio is equal to or less than the detection limit when an inductively coupled plasma mass spectrometer is used.

Fig. 2 is a graph showing a temporal change of hydrogen generated by reactions of the composite materials 100 of Exampled 1 and 2 and the silicon particles of a comparative example with a water-containing liquid.

As shown in Fig. 2, it was revealed that for the composite material 100 of Example 1, a large amount of generation of hydrogen (591 mL (milliliter)/g (gram)), and a very large amount of hydrogen per unit mass (mL/g) immediately after the generation of hydrogen (particularly within 2 to 3 hours after generation of hydrogen) was obtained. Specifically, the amount of generation of hydrogen until 3 hours after generation of hydrogen was a very large value of about 385 mL/g. It is worth noting that a high capability of generating hydrogen can be exhibited over a long time of 40 hours or more (at least 48 hours in an example) after the start of generation of hydrogen as in Example 1.

In addition, it was revealed that for the composite material 100 of Example 2, a relatively large total amount of generation (368 mL/g), and a relatively large amount of hydrogen per unit mass (mL/g) immediately after the generation of hydrogen (particularly within 2 to 3 hours after generation of hydrogen) was obtained. Specifically, the amount of generation of hydrogen until 3 hours after generation of hydrogen was a relatively high value of about 170 mL/g. It is worth noting that a high capability of generating hydrogen can be exhibited over a long time of 40 hours or more (at least 48 hours in an example) after the start of generation of hydrogen as in Example 2.

As shown in Fig. 2, it was revealed that the comparative example in which the supporting step in the first embodiment had not been carried out showed an amount of generation of hydrogen (309 mL/g) smaller than that for the composite material 100, and the smallest amount of hydrogen per unit mass (mL/g) immediately after the generation of hydrogen (particularly within 2 to 3 hours after generation of hydrogen). Specifically, the amount of generation of hydrogen until 3 hours after generation of hydrogen was a low value of about 75 mL/g.

In order to confirm the reproducibility, the inventor of the present application examined the amount of hydrogen per unit mass (mL/g) for cases where iron (Fe) was employed as the metal element 20 as in Example 1, and the mass ratio of the metal element 20 to silicon particles 10 was set to about 0.5 ppmw, about 1.5 ppmw and about 2.0 ppmw, and the results showed that in each of the cases, the amount of hydrogen per unit mass (mL/g) (i.e. rate of generation of hydrogen) immediately after generation of hydrogen (particularly within 2 to 3 hours after generation of hydrogen) showed a value higher than that in the comparative example. Interestingly, in each of the above-described three types of mass ratios, values similar enough to be considered as falling within an experimental error range were obtained for the amount of hydrogen per unit mass (mL/g) immediately after generation of hydrogen and the amount of generation of hydrogen (mL/g) until completion of the hydrogen generation reaction.

### <Analysis with transmission electron microscope (TEM) image>

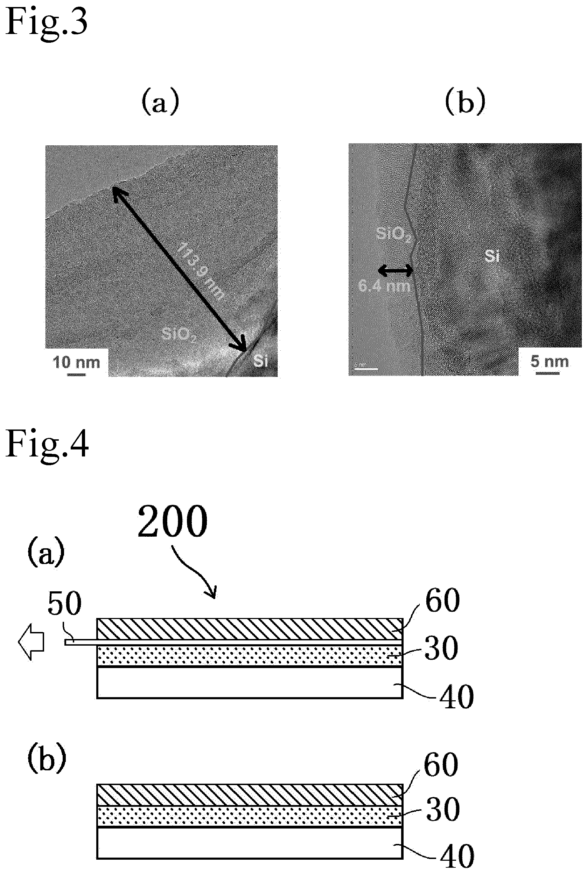

Further, using a transmission electron microscope (TEM), the present inventor analyzed the surface-covering silicon oxide film formed by the reaction between the composite material 100 of Example 1 and the water-containing liquid and the reaction between the silicon particles of the comparative example and the water-containing liquid. Fig. 3 shows cross-sectional TEM images showing silicon oxide thicknesses for the composite material (a) of Example 1 and the comparative example (b).

Very interestingly, from comparison between Figs. 3(a) and 3(b), it was confirmed that the thickness of the silicon oxide of the composite material 100 of Example 1 (about 113.9 nm) was 17 times or more the thickness of the silicon oxide of the comparative example (about 6.4 nm). At present, the detailed mechanism is not clear, but it is considered that a part of the metal element 20 on the surfaces of the silicon particles 10 exhibit a catalytic effect. It is considered that resultantly, as compared to a case where the metal element 20 is not present, the hydrogen generation reaction can be promoted, resulting in generation of a situation in which the thickness of silicon suboxide and/or the thickness of the mixed composition of silicon suboxide and silicon dioxide are easily increased (in other words, the hydrogen generation reaction is likely to occur).

Therefore, when a composite material is employed in which the metal element 20 is supported on or attached or adsorbed to at least a part of surfaces of silicon particles 10, or the surfaces are chemically bonded to the metal element 20, the thickness of silicon suboxide covering at least a part of the surfaces of the silicon particles 10 and/or the thickness of the mixed composition of silicon suboxide and silicon dioxide are larger than the thickness for conventional silicon fine particles. The large thickness indicates that the reaction in which silicon and water (or water in a water-containing liquid) contact each other to form silicon oxide and hydrogen can more reliably occur as compared with conventional silicon fine particles.

As described above, employment of the composite material 100 in which at least a part of surfaces of silicon particles 10 support the metal element 20 of which mass ratio to silicon particles 10 is in the numerical value range of at least 0.1 ppmw or more and 1000 ppmw or less or the surfaces are chemically bonded to the metal element 20 in the above-mentioned numerical value range can significantly contribute to more intensive exhibition of the capability of generating hydrogen of the composite material 100. For example, it is possible to significantly contribute to generating a large amount of hydrogen rapidly and over a long period of time depending on a situation or more reliably extracting a large amount of hydrogen in a human or animal body, or in various moisture-containing spaces or water-containing liquids.

### <Modification (1) of first embodiment>

An aspect in which a reforming step of bringing the surfaces of silicon particles into contact with hydrogen peroxide to reform the surfaces is carried out after the pulverization step is carried out and before the supporting step is carried out in the first embodiment is also a preferred aspect. By this reforming step, silicon particles including silicon nanoparticles can be made hydrophilic in a macroscopic point of view. For example, the reforming step can be carried out by immersing silicon particles in hydrogen peroxide water contained in a known container (about 10°C to about 80°C, and about 20°C to about 50°C from the viewpoint of further reducing the cost).

From the experiments by the present inventor, it has been confirmed that the amount of generation of hydrogen with the composite material 100 according to the first embodiment (in which the reforming step using hydrogen peroxide water was not carried out) is larger than the amount of generation of hydrogen under conditions where the reforming step using hydrogen peroxide water is carried out and the supporting step in the first embodiment is not carried out.

The same reforming can be performed by immersing the silicon particles in ozone water and/or sodium percarbonate instead of hydrogen peroxide water. Alternatively, the same reforming can be performed by bringing the silicon particles into contact with at least one selected from the group consisting of hydrogen peroxide water, ozone water and sodium percarbonate.

As described above, it is also preferred to carry out the reforming step using hydrogen peroxide water at substantially room temperature from the viewpoint of performing treatment at low cost and safely. In addition, employment of hydrogen peroxide water in the reforming step of the present modification is a preferred aspect in that hydrogen can be generated by using a safer and more secure material (e.g. having less influence on the human body) as in the case of ethanol.

### <Modification (2) of first embodiment>

In the present modification, 500 mg of the composite material 100 according to the first embodiment or the modification (1) of the first embodiment is mixed with about 500 mg of sodium hydrogencarbonate powder (manufactured by Wako Pure Chemical Industries, Ltd., purity: 99.5%). The mixture can be kneaded, and formed into a tablet (e.g. a tablet for medicaments or quasi-pharmaceutical products) as a columnar lump body having a diameter of about 8 mm and a height of about 4 mm by a tableting method. The tablet is an example of a lump preparation. An aspect in which the composite material 100 having stable silicon suboxide and a pH adjusting agent such as sodium hydrogencarbonate are separately formed into nanocapsules, microcapsules or normal capsules insoluble under acidic conditions and soluble under basic conditions, or applied is a preferred aspect. By employing the aforementioned aspect, the preparation can be dissolved to promote a reaction of the composite material 100 with water in the presence of moisture under basic conditions while avoiding reaction in the presence of moisture under acidic conditions.

### <Modification (3) of first embodiment>

The composite material 100 according to the first embodiment or each of the modifications (1) and (2) of the first embodiment can be applied as, for example, a preparation (medicament) or a quasi-pharmaceutical product. In addition, the application thereof is not limited to tablets. For example, even when a capsule preparation with the composite material 100 encapsulated in a capsule is employed in place of a tablet, the same effect as the above-mentioned effect can be exhibited. The composite material 100 can generate much hydrogen when the composite material is in the powdery form having a large surface area rather than being in a lump form, but the composite material is orally ingested or taken through the anus easily when formed into a tablet or a capsule preparation. In addition, when the composite material is formed into a tablet or a capsule preparation, it maintains a lump form to some extent, but is increasingly disintegrated to assume a powdery form after passing through the stomach. Thus, in the stomach where it is desired to suppress the hydrogen generation reaction, the surface area of the composite material exposed to gastric fluid and/or gastric contents can be reduced, and in the small intestine and/or large intestine where it is desired to promote the hydrogen generation reaction, the surface area of the composite material 100 exposed to the water-containing liquid can be increased.

In addition, the composite material 100 may be a granular preparation. The granular preparation assumes a powdery form in an earlier stage after being orally ingested or taken through the anus as compared to tablets and capsules. In the case of oral ingestion, however, since the pH value of gastric fluid is low (about 1.5), the preparation generates little hydrogen even when assuming a powdery form immediately after reaching the stomach, and generates hydrogen in the presence of water after passage through the stomach.

The composite material 100 may be a powdered preparation. The powdered preparation is easy to handle when the composite material 100 is used as a constituent component of a food product such as a health food product, e.g. a food additive. When the composite material is used as a food additive, silicon fine particles 10 having a crystallite diameter of 1 nm or more and 10 µm or less or 1 nm or more and 500 nm or less (in a narrower sense, 1 nm or more and 100 nm or less) may be mixed and used as the composite material 100. Preferably, the silicon fine particles 10 are contained in an amount of 1 mass% or more. The upper limit of the content of silicon fine particles 10 is not specified, but is preferably 40 mass% or less with consideration given to the taste.

In addition, an example of a covering layer applicable to a tablet is a known enteric material hardly soluble in the stomach, which is a coating agent that covers the outermost layer of the tablet. In addition, an example of a covering layer applicable to a capsule preparation is a capsule itself which encapsulates the composite material 100, and is produced from a known enteric material hardly soluble in the stomach.

As described above, an example of a preparation suitable as an application of the composite material 100 is a tablet which is a lump preparation which is easily orally ingested or taken through the anus in a sufficient amount, or a capsule preparation in which the powdery composite material 100 (which may include those formed into aggregates) are encapsulated in a capsule. When a tablet is employed, a disintegrating agent may be further included. For the disintegrating agent, a known material can be employed. In addition, an example of a more preferred disintegrating agent is an organic acid, and the most preferred example is citric acid. Here, the organic acid can also function as a binding agent that brings silicon particles 10 into a lump form. The composite material 100 can also be applied as, for example, granular, flaky and/or powdered food topping materials (typically, "rice seasoning") for each food item.

### <Modification (4) of first embodiment>

In addition, the composite material 100 according to the first embodiment or each of the modifications (1) to (3) of the first embodiment enables hydrogen to be taken into the body transdermally or transmucosally (including the skin itself or the mucosa itself) by, for example, using a "medium" that is brought into contact with the composite material 100. The material or the commercial product for the medium in the present modification is not particularly limited. As long as the medium is physiologically acceptable, the effects of the present modification can be exhibited. Therefore, a material including the composite material 100 and the medium that contacts the composite material 100 can exhibit a function as a hydrogen supply material.

Specifically, from the viewpoint of increasing the opportunity for a portion of a human to be brought into contact with water (or a water-containing liquid) or a medium containing the water (or the water-containing liquid) (hereinafter, also referred to collectively as a "medium") in a living scene, an example of a preferred medium is in the form of at least one selected from the group of liquid, gel, cream, paste, emulsion and mousse. Another example of a preferred medium is bathing water (bathing water which is preferably alkaline). Therefore, in an example of the present modification, the production of the bathing water is a method for producing the medium.

Bath water will be described in more detail. Typically, tap water is stored as bath water in a common bath (a bathtub in a public bathhouse, a public bathtub, and an indoor or outdoor bathtub installed by an inn). Before and after the bath water is stored, the composite material is placed or put in the bathtub, and a contact step of bringing the composite material 100 into contact with the bath water as a medium is carried out to generate hydrogen (H₂). Therefore, the composite material 100 of the present modification can be employed as a so-called bath agent.

Therefore, the hydrogen (H₂) generated by the contact step can be brought into contact with the skin and/or a mucous membrane of the bathing human via bathing water as a physiologically acceptable medium. As a result, according to the present modification, it is possible to take hydrogen (H₂) into the human body (including the skin itself or the mucous membrane itself) using means different from oral ingestion or uptake through the anus.

### SECOND EMBODIMENT

In addition, for the composite material 100 according to the first embodiment or each modification thereof, for example, a layered body obtained by forming the composite material 100 in layered shape, or a layered body including the composite material 100 in a base material formed in a layered shape is also another aspect that can be employed. Therefore, the layered body can exhibit a function as a hydrogen supply material.

In the present embodiment, a laminated structure 200 of a layered body and a medium can be formed by using a layered body 30 obtained by forming the composite material 100 in a layered shape, or a layered body 30 including the composite material 100 in a base material formed in a layered shape. Fig. 4(a) is a side view showing the laminated structure 200 of a layered body and a medium before hydrogen is generated, and Fig. 4(b) is a side view showing the laminated structure 200 of a layered body and a medium when hydrogen is generated.

As shown in Figs. 4(a) and 4(b), the laminated structure 200 includes at least the layered body 30 and a medium 60 on or above a base portion 40 (e.g. fiber, natural resin, synthetic resin, metal, semiconductor, ceramic or glass). A preferred example of the medium 60 is a physiologically acceptable material and is in the form of at least one selected from the group of liquid, gel, cream, paste, emulsion and mousse. The medium 60 can contain a pH adjusting agent typified by sodium hydrogencarbonate. The base portion 40 having elasticity is a preferred aspect. In addition, particularly when the laminated structure 200 of the layered body 30 and the medium 60 can be held even if the base portion 40 is not provided, it is not necessarily required to provide the base portion 40.

Before hydrogen is generated, a film impermeable to water 50 is provided between the layered body 30 and the medium 60 so that the layered body 30 and the medium 60 are not brought into contact with each other as shown in Fig. 4(a). A film formed of a known material impermeable to water can be applied as the film 50. For example, an example of the material for the film impermeable to water 50 is a known polymer such as polyethylene. In addition, as another example, use of a water-soluble and water-impermeable sheet disclosed in WO 2011/036992 is also an aspect that can be employed.

On the other hand, as shown in Fig. 4(b), at least a part of the layered body 30 and the medium 60 is brought into direct contact with each other when the film 50 is drawn out in the arrow direction. As a result, collaboration with a pH adjusting agent typified by, for example, sodium hydrogencarbonate enables hydrogen to be generated when the layered body 30 is brought into the medium 60 which may contain a water-containing liquid having a pH value of 7 or more (more preferably more than 7, still more preferably more than 7.4).

In the present embodiment, the layered body 30 and the medium 60 are formed so as to directly contact each other when the film 50 is drawn out in the arrow direction (left direction in the drawing), and the method for removing the film 50 is not particularly limited. For example, an aspect in which the medium 60 is formed so as to be brought into contact with the composite material 100 (the layered body 30 in the present embodiment) when at least a part of the film 50 is removed or dissolved is an aspect that can be employed. Regarding an example of a material for dissolving at least a part of the film 50, for example, employment of a water-soluble and water-impermeable sheet disclosed in WO 2011/036992 is also an aspect that can be employed.

As another aspect, an aspect in which instead of the layered body 30, the composite material 100 according to the first embodiment or each of the modifications thereof is covered with the film impermeable to water 50 before generation of hydrogen can also be employed. If the composite material 100 and the medium 60 are at least partially brought into direct contact with each other when the film 50 is removed or dissolved, the same effect as in the case of the layered body 30 can be exhibited.

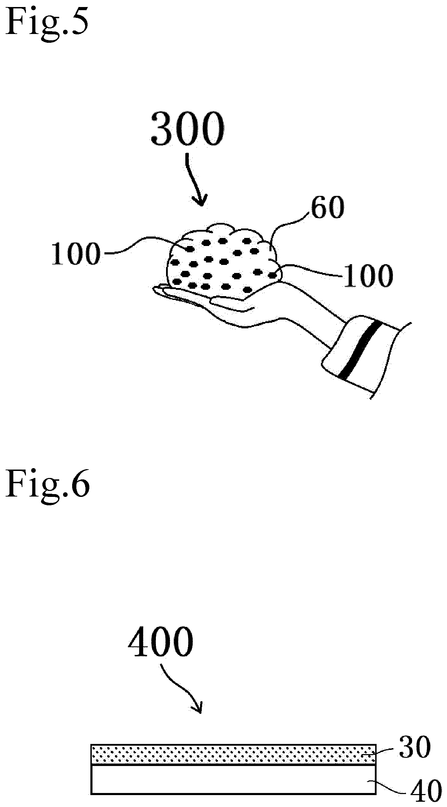

In addition, for example, when the medium is in the form of at least one selected from the group of liquid, gel, cream, paste, emulsion and mousse, there is the possibility that two layers (layered body 30 and medium 60) as shown in Fig. 4(b) do not maintain a clearly separated state. Rather, such a case is preferable from the viewpoint of more reliably promoting generation of hydrogen because the contact area between the composite material 100 and the medium 60 increases. For example, Fig. 5 is a schematic view showing a foam 300 containing or provided with the composite material 100. As shown in Fig. 5, an aspect in which a face washing foam component formed by sprayed from a known face washing spray can forms the medium 60 and the foam component contains or is provided with the composite material 100 is another preferred aspect. The spray can being formed so as not to contact moisture in the air is a further preferred aspect. In addition, an aspect in which the medium contains a physiologically acceptable pressure sensitive adhesive for bonding to, for example, a portion of a human is also a preferred aspect that can be employed.

### <Modification (1) of second embodiment>

As one modification of the second embodiment described above, the layered body 30 obtained by forming the composite material 100 in a layered shape may be employed as either the layered body 30 alone or a laminated structure of the base portion 40 and the layered body 30. The structure 400 as an example shown in Fig. 6 includes a layered body 30 on the base portion 40. When the shape of a layered body can be held even if the base portion 40 is not provided, it is not necessarily required to provide the base portion 40. In addition, from the viewpoint of avoiding contact with moisture in the air with high reliability, the film impermeable to water 50 may be provided so as to cover the layered body 30.

In addition, as shown in Fig. 6, for example, an aspect in which after the layered body 30 is brought into contact with the human skin or a mucous membrane, sweat or body fluid containing moisture from the skin or a mucous membrane is brought into contact with the layered body 30 to generate hydrogen is another preferred aspect of the present embodiment. Such an aspect also enables a human to take hydrogen into the body. For moisture, water (clean water or the like) can be supplied by spraying or the like before (e.g. immediately before) the layered body 30 is used instead of sweat or body fluid.

It is worth noting that the structure or laminated structure that can be employed in the second embodiment and the modification (1) thereof is a structure that can be employed in various "living scenes". For example, typical examples of commercial products which may employ (include) the medium are the following (A) to (D).
(A) One cleaning agent selected from the group of a facial wash, a hair shampoo, a body shampoo, a liquid hand soap and a liquid body soap.
(B) One cosmetic material selected from the group of a beauty lotion (e.g. one containing hyaluronic acid), a serum, an emulsion, a lotion, a cosmetic cream (e.g. one containing collagen), a foundation, a skin pack (including a skin pack having a gel (or gel-like agent)), a shaving cream, a hair rinse, a hair treatment, a hair conditioner, a hair cosmetic, an antiperspirant, and an UV protecting cosmetic.
(C) One therapeutic material selected from the group of an ointment and a plaster.
(D) One hygienic material selected from the group of a water-absorbent resin, a water-absorbent nonwoven fabric, a water-absorbent fiber, a water-absorbent felt and a water-absorbent gel (or gel-like agent).

Here, the "hair cosmetics" include hairdressing materials, hair oils, camellia oil, styling agents (materials), set agents (materials), blowing agents (materials), brushing agents (materials), tics, hair sticks, hair waxes, hair foams, hair gels, pomades, hair creams, hair solids, hair lacquers, hair liquids, hair sprays, and hair water. In addition, the "sanitary materials" include sanitary gloves, head covers, headbands, bed pads, bed sheets, adult incontinence products, menstrual items, clothing items, wound dressings (including wound dressing materials, tapes and bandages), disposable diapers including adult diapers and pediatric diapers, gauzes, gowns, sanitary tissues (including towels, facecloths, patches, wet tissues, and napkins), absorbent cotton, cotton swabs, adhesive plasters and surgical tapes.

### ANOTHER EMBODIMENT (1)

Further, the composite material 100 of each of the above-described embodiments or modifications thereof can also be used as, for example, feed for animals for raising (in the present application, including raising in ranches) (which include dogs, cats, horses, sheep, rabbits or chickens and do not including fish), animals for food, animals whose hair or skin can be used for clothing, leather products (including pouches, various cases or bags) or the like (which include foxes, bears, deer, snakes or alligators), animals applied for medical use, fish including fish for aquaculture, or the like. Further, the solid preparation can be used as an industrial chemical or agent.

The composite material 100 according to each of the embodiments and modifications thereof can also be used as a human supplement or food additive. It is worth noting that the composite material according to each of the embodiments or modifications thereof has a capability of generating hydrogen and thus can exhibit a preservative function for various food products or materials. For example, when fresh food products including vegetables, fruits, fresh fish and fresh meat are brought into contact with water including the composite material 100 or water having the composite material 100 immersed therein, it is possible to improve the shelf lives of the fresh food products. In addition, when the composite material 100 is immersed in various cosmetics or various perfumery containing perfume water, emulsion, cream or beauty lotion containing water (moisture), it is possible to improve the shelf lives of the cosmetics or perfumery.

### ANOTHER EMBODIMENT (2)

In addition, the composite material according to each of the embodiments or modifications thereof can be aggregated in a natural state to form aggregates having a diameter size of µm order (e.g. several tens of µm or more). The composite material 100 can be artificially put together by addition of the aggregate or a binding agent, compression or the like to form a formulation as a lump solid preparation having such a size that it can be picked up by human fingers. The formulation can also be applied to plants (including trees).

Specifically, in the present embodiment, the formulation is embedded in soil (containing moisture) in which the plant is planted or is grown naturally. In this way, the soil is applied as a medium containing a water-containing liquid. The formulation is brought into contact with soil as a medium to generate hydrogen (H₂). As a result, the plant contacting the soil can take hydrogen into the body through its root, stem or outer skin. As a result, inhibition of photosynthesis, decoloration of leaves, promotion of growth, and/or prevention or suppression of plant death can be achieved. In addition, for some plants, the sugar content can also be improved. A typical example of the moisture in the present embodiment is rainwater or artificial water. The number or amount of the formulation in the soil is not particularly limited.

In addition, an aspect in which the formulation is introduced or pot into a naturally occurring or artificial pool of water (medium) to bring the formulation and the water-containing liquid into contact with each other can also be employed as another aspect of the present embodiment. The formulation and the water-containing liquid are brought into contact with each other to generate hydrogen (H₂). In this aspect, an animal can take hydrogen into the body through the water-containing liquid when coming into contact with the pool of water or immersing itself in the pool of water. As a result, hydrogen taken into the body directly, transdermally or transmucosally can appropriately eliminate, remove or reduce excessive active oxygen (particularly hydroxyl radicals) in the body of the animal, so that health promotion and/or disease prevention of the animal can be performed.

In addition, if the pH value of the pool of water is above that of the weak acidity (e.g. the pH value is 5 or more), the pH value is made high by using a mixture of the formulation in the present embodiment and sodium hydrogencarbonate, so that it is possible to satisfy the condition as a medium with which hydrogen (H₂) is easily generated. In other words, when the water-containing liquid such as a pool of water is acidic, it is necessary to introduce or put a large amount of the mixture into the soil in order to satisfy the condition as a medium with which hydrogen (H₂) is easily generated.

When the mixture of the formulation and sodium hydrogencarbonate in the present embodiment is used, the embedded in, or introduced or put into the soil or the pool of water to carry out the contact step of bringing the composite material 100 into contact with a medium even if the soil or the pool of water as a medium is neutral or weakly acidic. As a result, the generation of hydrogen (H₂) can be promoted.

Therefore, via the soil or the pool of water as a medium, hydrogen (H₂) generated by the contact step can be brought into contact with the skin and/or a mucous membrane of an animal or the leaves, stems, skins and/or roots of plants. As a result, according to the present embodiment, it is possible to take hydrogen (H₂) into the body of an animal or a plant. For example, for animals, a function of suppressing aging can be exhibited. In addition, for plants, the hydrogen can exhibit a function of suppressing corrosion.

The present embodiment is not limited to a case where the composite material 100 or the formulation is used as it is. An aspect in which the composite material 100 or the formulation is contained in a "base material" such as, for example, a medicament for animals, a food product for livestock or pets, a feed for animals, a medicament for plants, a fertilizer for plants, or compost for plants, or blended in the "base material" is also a preferred aspect that can be employed. For example, it is a typical example that the composite material 100 or the formulation is mixed or kneaded as an additive at, for example, 0.1 wt% to 50 wt% in the base material. Therefore, in the present embodiment, not only a formulation obtained by blending the composite material 100 with a medicament for plants, a fertilizer for plants, or a compost for plants, but also the "base material" is a "formulation" in a broad sense. Thus, contact of the base material with the medium can be employed as one suitable means for the animal or plant to take hydrogen into the body, for example, transdermally or transmucosally.

### ANOTHER EMBODIMENT (3)

For example, the composite material 100 according to each of the embodiments and modifications thereof is brought into contact with a first water-containing liquid having a pH value of less than 7 in an initial contact step (a first contact step), and brought into contact with a second water-containing liquid having a pH value of 7 or more in a subsequent contact step (second contact step). In this way, hydrogen can be generated in the second contact step. The composite material 100 according to each of the embodiments can have a significant capability of generating hydrogen when coming into contact with a water-containing liquid having a pH value of 7 or more (more preferably more than 7, still more preferably 8.2 or more).

In addition, the temperature condition of the second water-containing liquid for generation of hydrogen is not limited. Possibly depending on the pH of the second water-containing liquid, generation of hydrogen can be promoted with high reliability as long as the temperature of the second water-containing liquid is 80°C or lower. However, the upper limit of the temperature of the second water-containing liquid is not limited. For example, when the composite material 100 according to each of the embodiments and modifications thereof is used as an industrial chemical, the temperature may exceed 50°C. However, when the temperature becomes higher, there is the problem that equipment (including a container) is required to have high heat resistance, and care is needed for handling. Therefore, the temperature is preferably 100°C or lower even when the solid preparation is used as an industrial chemical.

As described above, the disclosure of each of the embodiments and modifications thereof is intended for explanation of those embodiments or modifications, and is not intended to limit the present invention. In addition, claims also include other modifications within the scope of the present invention including other combinations of the embodiments and modifications thereof.

### INDUSTRIAL APPLICABILITY

The composite material of the present invention can be widely used in various industries including agriculture industry, agriculture and stock raising industry, forestry industry, fishery industry, pet industry, industries involving potted plants and fresh flowers, pharmaceutical industry (including quasi-pharmaceutical products) and medical industry, food industry, veterinary industry and tree health industry, cosmetics industry, industries involving industrial reducing agents, rust prevention applications and synthesis processes for industrial chemicals, and new energy industries involving fuel cells and the like.

### DESCRIPTION OF REFERENCE SIGNS

10: Silicone particles, silicon fine particles
20: Metal element
30: Layered body
40: Base portion
50: Film impermeable to water
60: Medium
100: Composite material
200: Laminated structure
300: Foam containing or provided with the composite material
400: Structure

## Claims

1. A composite material comprising:
silicon particles or silicon fine particles capable of generating hydrogen; and
iron (Fe) supported on, or attached or adsorbed to at least a part of surfaces of the silicon particles or silicon fine particles, or chemically bonded to the surfaces.

2. The composite material according to claim 1, wherein a mass ratio of the iron (Fe) is 0.1 ppmw or more and 1000 ppmw or less when the silicon particles or the silicon fine particles is assumed to be 1.

3. The composite material according to claim 1, wherein a mass ratio of the iron (Fe) is 0.5 ppmw or more and 1000 ppmw or less when the silicon particles or the silicon fine particles is assumed to be 1.

4. The composite material according to any one of claims 1 to 3, wherein a ratio of the silicon fine particles having a crystallite diameter of less than 1 µm and aggregates of the silicon fine particles having a crystallite diameter of less than 1 µm to all the silicon particles, the silicon fine particles and the aggregates thereof is 5 mass% or less.

5. A medicament comprising the composite material according to any one of claims 1 to 4.

6. A quasi-pharmaceutical product comprising the composite material according to any one of claims 1 to 4.

7. A hydrogen supply material comprising:
the composite material according to any one of claims 1 to 4; and
a physiologically acceptable medium contacting the composite material,
the hydrogen supply material bringing hydrogen produced from the composite material into contact with the skin and/or a mucous membrane via the medium.

8. The hydrogen supply material according to claim 7, further comprising a film impermeable to water, wherein the film covers the composite material or covers a layer including the composite material,
wherein the medium is brought into contact with the composite material when at least a part of the film is removed or dissolved.

9. The hydrogen supply material according to claim 7 or 8, wherein the medium is in the form of at least one selected from the group of liquid, gel, cream, paste, emulsion and mousse.

10. A feed comprising the composite material according to any one of claims 1 to 4.

11. The feed according to claim 10 for animals (excluding fish).

12. The feed according to claim 10 for fish.

13. A supplement comprising the composite material according to any one of claims 1 to 4.

14. A food additive comprising the composite material according to any one of claims 1 to 4.

15. A food product comprising the composite material according to any one of claims 1 to 4.

16. A formulation obtained by blending the composite material according to any one of claims 1 to 4 with a medicament for plants, a fertilizer for plants, or a compost for plants.

17. A fresh food product, a cosmetic or perfumery, which has been brought into contact with water having the composite material according to any one of claims 1 to 4 immersed therein, or water including the composite material.
